# EUROPÄISCHE PATENTSCHRIFT

(11) **EP 0 786 250 B1**
(45) Veröffentlichungstag und Bekanntmachung des Hinweises auf die Patenterteilung: **02.04.2003**
(21) Anmeldenummer: 97100586.3
(22) Anmeldetag: 16.01.1997
(51) Int. Cl.: A61K 7/50, A61K 7/06

(54) **Haaravivierende Zubereitungen**
Composition for reviving hair
Composition d'avivage pour la chevelure

(30) Priorität: 23.01.1996 DE 19602242
(43) Veröffentlichungstag der Anmeldung: 30.07.1997
(73) Patentinhaber: Henkel Kommanditgesellschaft auf Aktien, 40589 Düsseldorf-Holthausen (DE)
(72) Erfinder: Priebe, Christian, 42489 Wülfrath (DE); Seidel, Kurt, 40589 Düsseldorf (DE); Hollenberg, Detlef, Dr., 40699 Erkrath (DE); Goddinger, Dieter, Dr., 25436 Tornesch (DE)

(56) Entgegenhaltungen:
- EP-A- 0 423 894
- EP-A- 0 636 356
- WO-A-88/03016
- US-A- 4 976 956
- US-A- 5 525 245

## Beschreibung

### Gebiet der Erfindung

Die Erfindung betrifft avivierende Zubereitungen enthaltend sogenannte Esterquats und Alkylamidoamine. Das Wirkstoffgemisch weist synergistische Eigenschaften auf und zeigt eine gute biologische Abbaubarkeit. Die Kombination der Wirkstoffe bewirkt beispielsweise beim menschlichen Haar eine hervorragende Naßkämmbarkeit bei gleichzeitig erhöhtem Trockenvolumen und verringerter elektrostatischer Aufladungsbereitschaft der Haare.

### Stand der Technik

Menschliches Haar wird heute in vielfältiger Weise mit haarkosmetischen Zubereitungen behandelt. Dazu gehören etwa die Reinigung der Haare sowie das Bleichen, Färben und Verformen der Haare mit Wellmitteln, Tönungsmitteln und Stylingpräparaten. Sowohl der Gebrauch von Shampoos als auch die dekorative Gestaltung der Frisur beispielsweise durch Färben oder Dauerwellen sind Eingriffe, die die natürliche Struktur und die Eigenschaften der Haare beeinflussen. So können anschließend an eine solche Behandlung beispielsweise die Naß- und Trockenkämmbarkeit des Haares, der Halt und die Fülle des Haares unbefriedigend sein oder die Haare einen erhöhten Spliß aufweisen.

Es hat daher nicht an Ansätzen gefehlt, die Haare mit entsprechenden Wirkstoffen nachzubehandeln, um die erwähnten Umstände in ihrem Ausmaß zu mildem oder zu beseitigen. In der Regel wurden dazu kationische Tenside herangezogen, häufig in Kombination mit weiteren Hilfsstoffen. Eine solche Nachbehandlung wird vorzugsweise in Form einer Spülung oder Haarkur angewandt, die als Wirkstoff beispielsweise quartäre Ammoniumsalze oder spezielle Polymere beinhaltet. Dieses aus dem Stand der Technik weithin bekannte Vorgehen ist jedoch bislang in mehrfacher Hinsicht unbefriedigend.

Textilien und textile Gewebe sind während ihrer Verarbeitung, beim Gebrauch sowie der regelmäßigen Reinigung ständig belastenden äußeren Einflüssen ausgesetzt. Die damit einhergehenden Veränderungen bewirken ein Nachlassen der Weichheit des Gewebes und einen weniger weichen Griff. Es besteht also auch ein Marktbedürfnis nach Avivagemitteln für textile Fasern, die den Geweben einen verbesserten Weichgriff verleihen. Auch hier haben sich kationische Tenside prinzipiell als geeignete Wirkstoffe für Avivagemittel erwiesen.

Die üblicherweise bislang als kationische Tenside eingesetzten quartären Ammoniumverbindungen sind aber nur unzureichend biologisch abbaubar und teilweise ökotoxisch, so daß ihr Einsatz aus ökologischen Gründen möglichst verringert werden sollte. Weiterhin besitzen diese Ammoniumverbindungen ein zum Teil erhebliches Irritationspotential für die Haut.

Es bestand daher Bedarf nach Faserbehandlungsmitteln, die eine avivierende Wirkung aufweisen und deren Inhaltsstoffe gute biologische Abbaubarkeit sowie ein möglichst geringes oder kein Irritationspotential und eine geringe Ökotoxizität aufweisen. Weiterhin sollen die in solchen Mitteln eingesetzten Inhaltsstoffe in möglichst geringen Mengen eine ausreichende Wirksamkeit entfalten, um Anwender und Umwelt zu schonen.

Überraschend wurde nun gefunden, daß die Kombination aus sogenannten Esterquats und Alkylamidoaminen eine hervorragende faseravivierende Wirkung zeigt und durch synergistische Effekte zu einer Einsparung an Wirkstoffen führt. Die Behandlung menschlicher Haare mit den erfindungsgemäßen Mitteln, die eine solche Wirkstoffkombination enthalten, führt zu einer guten Naß- sowie vorteilhaften Trockenkämmbarkeit bei gleichzeitig gutem Frisurenhalt und geringer Neigung zu statischer Aufladung. Gleichzeitig zeichnen sich diese beiden Wirkstoffgruppen durch gute biologlische Abbaubarkeit und ein geringes Irritationspotential aus.

Die Verwendung von Esterquats in Avivagemitteln ist bekannt. So beschreibt beispielsweise die DE-A1-41 38 630 saure Haarpflegemittel mit Fettsäuretrialkanolaminestersalzen als Wirkstoff. Der synergistisch wirkende Zusatz von Alkylamidoaminen wird in dieser Anmeldung nicht erwähnt.

Die Avivageeigenschaften von Esterquats werden ebenso in der DE-A1-43 09 568 erwähnt. Die Druckschrift beschreibt den Einsatz von Esterquats in Haarpflegemitteln zusammen mit Polyhydroxyfettsäureamiden. Eine synergistische Wirkung wird nicht erwähnt.

Die DE-C1-44 02 852 lehrt den Einsatz von Esterquats in Verbindung mit schmutzablösenden Polymeren in Detergensgemischen zur Herstellung von Wäscheweichspülem. Die Verwendung von Alkylamidoaminen zur Verbesserung der avivierenden Eigenschaften sowie zur Verringerung der Wirkstoffmenge wird nicht vorgeschlagen.

Die EP-B1-0 292 481 lehrt die Verwendung quartärer Ammoniumsalze zusammen mit Alkylamidoaminen in Haarbehandlungsmitteln. Weder wird in dieser Druckschrift die Verwendung der erfindungsgemäßen Esterquats vorgeschlagen oder nahegelegt, noch ist der Druckschrift ein Hinweis auf die Existenz eines synergistischen Effekts zu entnehmen.

### Beschreibung der Erfindung

Gegenstand der Erfindung sind daher wäßrige Zubereitungen zur avivierenden Faserbehandlung, dadurch gekennzeichnet, daß sie eine Wirkstoffkombination, bestehend aus 0,01 bis 30 Gew.-% Esterquats, 0,01 bis 30 Gew.-% Alkylamidoaminen und 0,01 bis 15 Gew.-% an Fettalkoholen mit 8 bis 22 C-Atomen, jeweils bezogen auf die gesamte Zusammensetzung, enthalten.

Bei den erfindungsgemäßen Esterquats handelt es sich um bekannte Stoffe, die man nach den einschlägigen Methoden der präparativen organischen Chemie erhalten kann. In diesem Zusammenhang sei auf die Internationale Patentanmeldung WO 91/01295 verwiesen, nach der man Triethanolamin in Gegenwart von unterphosphoriger Säure mit Fettsäuren partiell verestert, Luft durchleitet und anschließend mit Dimethylsulfat oder Ethylenoxid quatemiert. Stellvertretend für den umfangreichen Stand der Technik sei an dieser Stelle auf die Druckschriften US 3,915,867, US 4,370,272, EP-A2 0 239 910, EP-A2 0 293 955 A2, EP-A2 0 295 739 und EP-A2 0 309 052 verwiesen. Übersichten zu diesem Thema sind beispielsweise von R.Puchta et al. in *Tens.Surf.Det*., 30, 186 (1993), M.Brock in *Tens. Surf. Det*., 30, 394 (1993) und R.Lagerman et al. in *J.Am.Oil.Chem.Soc*., 71, 97 (1994) erschienen.

Die quaternierten Fettsäuretriethanolaminestersalze haben die Formel **(I)**, 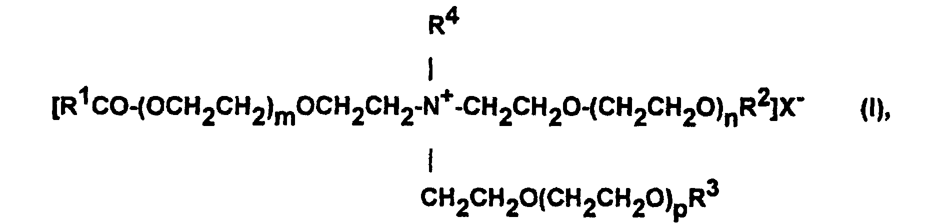 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

Typische Beispiele für Esterquats, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielweise bei der Druckspaltung natürlicher Fette und Öle anfallen.

Vorzugsweise werden technische C_{12/14}- und C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt.

Zur Herstellung der quatemierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}- Talg- bzw. Palmfettsäure (lodzahl 0 bis 40) ab.

Aus anwendungstechnischer Sicht haben sich quatemierte Fettsäuretriethanolamin-estersalze der Formel **(I)** als besonders vorteilhaft erwiesen, in der R¹CO für einen Acylrest mit 16 bis 18 Kohlenstoffatomen, R² für R¹CO, R³ für Wasserstoff, R⁴ für eine Methylgruppe, m, n und p für 0 und X für Methylsulfat steht.

Neben den quatemierten Fettsäuretriethanolaminestersalzen kommen als Esterquats ferner auch quatemierte Estersalze von Fettsäuren mit Diethanolalkylaminen der Formel **(II)** in Betracht, 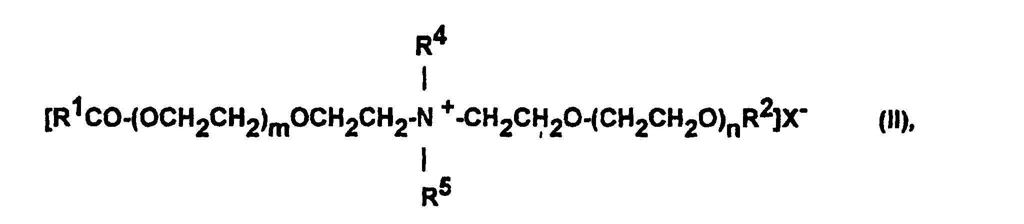 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Als weitere Gruppe geeigneter Esterquats sind schließlich die quaternierten Estersalze von Fettsäuren mit 1,2-Dihydroxypropyldialkylaminen der Formel **(III)** zu nennen, 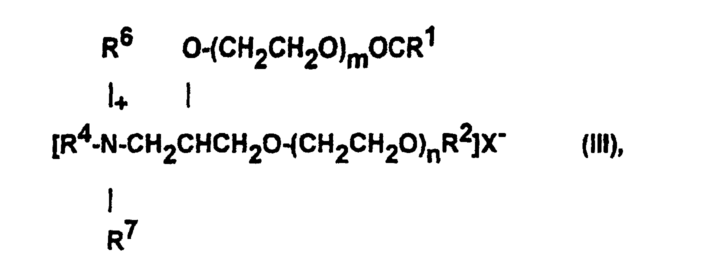 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Hinsichtlich der Auswahl der bevorzugten Fettsäuren und des optimalen Veresterungsgrades gelten die für **(I)** genannten Beispiele auch für die Esterquats der Formeln **(II)** und **(III)**. Üblicherweise gelangen die Esterquats in Form 50 bis 90 Gew.- %iger alkoholischer Lösungen in den Handel, die bei Bedarf problemlos mit Wasser verdünnt werden können.

Die anhand der Formeln **I** bis **III** beschriebenen Substanzen stellen naturgemäß Einzelstoffe dar, wobei für den erfindungsgemäßem Einsatz in der Regel auch Gemische dieser Stoffe infrage kommen können. Ebenso möglich ist gegebenenfalls der Einsatz der technischen Stoffgemische, wie sie aufgrund der Herstellungsverfahren in wechselnder Zusammensetzung erhältlich sind.

Neben den Esterquats enthält das Wirkstoffgemisch Alkylamidoamine der allgemeinen Formel 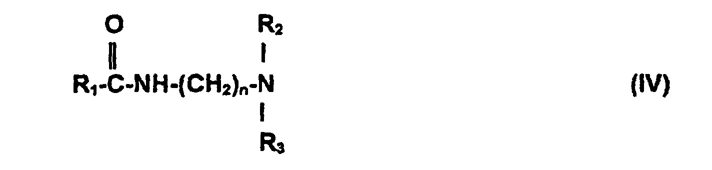 als synergistisch wirkende Komponente.

Hierbei steht **R**_{**1**}**CO** für einen aliphatischen, linearen oder verzweigten Acylrest mit 6 bis 22 Kohlenstoffatomen und 0, 1, 2 oder 3 Doppelbindungen, der in der Regel aus einer natürlich vorkommenden oder synthetisch hergestellten Fettsäure stammt.

**R**_{**2**} und **R**_{**3**} stellen gleiche oder unterschiedliche Alkylreste dar, die gegebenenfalls funktionelle Gruppen wie Hydroxygruppen, Estergruppen, Amin-, Amid- oder sonstige polare Substituenten tragen können, wobei jedoch in der Regel unsubstituierte Kohlenwasserstoffreste, die allenfalls eine oder mehrere Verzweigungen aufweisen, bevorzugt sind. Insbesondere bevorzugt sind jedoch kurzkettige Kohlenwasserstoffreste mit 1 bis 6 C-Atomen, wobei Ethyl- und Methylreste erfindungsgemäß ganz besonders bevorzugt sind.

Die Zahl n ist eine ganze Zahl von 1 bis 10, wobei Werte für n von 2 bis 7, insbesondere von 2 bis 4 bevorzugt sind.

Die Alkylaminoamine werden üblicherweise durch Amidierung natürlicher oder synthetischer Fettsäuren und Fettsäureschnitte mit Dialkylaminoaminen hergestellt. Typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind erfindungsgemäß solche Alkylamidoamine, die einen linearen Fettsäurerest mit 8 bis 22 C-atomen, insbesondere mit 14 bis 20 C-atomen aufweisen.

Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die erfindungsgemäße Zubereitung kann je nach Einsatzzweck diese beiden Wirkstoffkomponenten in unterschiedlichen Mengen enthalten. So können beispielsweise 0,1 bis 20 Gew.-% Esterquats neben 0,1 bis 20 Gew.-% Alkylamidamin vorliegen, wobei es jedoch bevorzugt ist, 0,5 bis 15 Gew.-% Esterquats neben 0,5 bis 15 Gew.-% Alkylamidoamin zur Faserbehandlung beispielsweise im textilen Bereich einzusetzen. Üblicherweise werden die Wirkstoffe zur Textilbehandlung jedoch in Konzentrationen von 5 bis 15 Gew.-% eingesetzt.

Bei der Behandlung keratinischer Fasern, insbesondere von menschlichem Haar, können weitaus geringere Konzentrationen zum Erfolg führen. Hier werden in der Regel Wirkstoffkonzentrationen (bezogen auf die Einzelkomponenten) von 0,1 bis 7 Gew.-%, bevorzugt 0,2 bis 5 Gew.-% und insbesondere bevorzugt 0,3 bis 3 Gew.-%, eingesetzt. Besonders gute Ergebnisse lassen sich bei Wirkstoffkonzentrationen von 0,2 bis 2 Gew.-% erzielen. Die Anwendung der erfindungsgemäßen Zubereitungen an keratinischen Fasern, insbesondere an menschlichen Haaren, ist jedoch nicht auf den Einsatz der Wirkstoffe in geringer Konzentration limitiert. Es können ebenso Konzentrate zur Anwendung kommen, in denen die Wirkstoffe jeweils in Anteilen von 2 bis 20 Gew.-%, bevorzugt 3 bis 14 Gew.-% und insbesondere in 5 bis 12 Gew.-%, vorliegen.

Üblicherweise sind Esterquats und Alkylamidoamine in einem Gewichtsverhältnis von 20 zu 1 bis 1 zu 20, insbesondere von 10 zu 1 bis 1 zu 10, in der erfindungsgemäßen Zubereitung enthalten. Je nach gewünschtem Effekt kann ein Mischungsverhältnis von 5:1 bis 1:5 oder sogar von 3:1 bis 1:3 bevorzugt sein.

Der pH-Wert der erfindungsgemäßen Zubereitung liegt bevorzugt zwischen 2 und 7, wobei Werte von 2,5 bis 5,5, insbesondere von 3 bis 5 besonders bevorzugt sind. Zur Einstellung dieses pH-Wertes kann praktisch jede für kosmetische Zwecke verwendbare Säure verwendet werden. Üblicherweise werden Genußsäuren verwendet. Unter Genußsäuren werden solche Säuren verstanden, die im Rahmen der üblichen Nahrungsaufnahme aufgenommen werden und positive Auswirkungen auf den menschlichen Organismus haben. Genußsäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt.

Neben den Esterquats und den Alkylamidoaminen sind im erfindungsgemäßen Mittel zur Faserbehandlung noch Fettalkohole mit 8 bis 22 C-Atomen enthalten.

Die eingesetzten Fettalkohole können gesättigt oder ungesättigt und linear oder verzweigt sein. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Palmitylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll.

Der Darstellungsweg des Fettalkohols spielt für den erfindungsgemäßen Einsatz keine Rolle. Die Fettalkohole stammen jedoch bevorzugt von Fettsäuren ab, wobei üblicherweise von einer Gewinnung aus den Estem der Fettsäuren durch Reduktion ausgegangen werden kann.

Besonders bevorzugt ist der Einsatz solcher Fettalkohole, die aus der Veresterung und Reduktion natürlich vorkommender Fettsäuren erhältlich sind.

Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen.

Im Sinne der Erfindung können auch solche gesättigten oder ungesättigten Fettalkohole zum Einsatz kommen, die aus der Veresterung und Reduktion von Derivaten natürlich vorkommender Fettsäuren erhältlich sind. So sind beispielsweise der Ricinenalkohol, der Elaidylalkohol oder der Pelargonalkohol erfindungsgemäß einsetzbar.

Die Fettalkohole werden in Mengen von 0,01 bis 15 Gew.-%, bevorzugt von 0,1 bis 10 Gew.-% und besonders bevorzugt von 0,3 bis 6 Gew.-%, bezogen auf die gesamte Zubereitung, eingesetzt.

Neben den beschriebenen Fettalkoholen können auch deren Alkoxylierungsprodukte in Mengen von 0,1 bis 15 Gew.-% zum Einsatz kommen, insbesondere die Ethoxylierungsprodukte der Fettalkohole mit 8 bis 22 C-Atomen. Besonders bevorzugt ist hierbei der Einsatz von ethoxylierten, unverzweigten Fettalkoholen mit 8 bis 20 C-Atomen, wie sie aus der Reduktion und anschließenden Ethoxylierung natürlich vorkommender Fette und Öle wie beispielsweise Kokosöl, Palmöl, Palmkemöl oder Sonnenblumenöl erhältlich sind.

Die erfindungsgemäßen Zubereitungen können gegebenenfalls noch ein oder mehrere weitere nichtionische Tenside enthalten. Typische Beispiele sind Alkylphenolpolyglycolether, Fettsäurepolyglycolester, Fettsäureamidpolygylcolether, Fettaminpolyglycolether, alkoxylierte Triglyceride, Alk(en)yloligoglucoside, Fettsäure-N-alkylglucamide, Proteinhydrolysate (insbesondere pflanzliche Produkte auf Sojabasis), Polyolfettsäureester, Zuckerester, Sorbitanester und Polysorbate. Sofern die nichtionischen Tenside Polyglycoletherketten enthalten, können sie eine konventionelle, vorzugsweise jedoch eine eingeengte Homologenverteilung aufweisen.

Weiterhin sind als Zusatzstoffe solche ausgewählt aus der Gruppe der Dialkylether in Sinne der Erfindung einsetzbar. Bevorzugt werden solche Dialkylether verwendet, die insgesamt zwischen 12 und 36 C-Atomen enthalten. In einer besonders bevorzugten Ausführungsform werden solche Dialkylether eingesetzt, die insgesamt zwischen 12 und 24 C-Atomen aufweisen.

Die erfindungsgemäß eingesetzten Dialkylether enthalten bevorzugt nur eine Ethergruppe, wobei jedoch sowohl symmetrische als auch unsymmetrische Dialkylether zum Einsatz kommen können.

Symmetrische lineare Dialkylether sind beispielsweise Di-n-octylether, Di-n-decylether, Di-n-nonylether, Di-n-undecylether und Di-n-dodecylether.

Außer den symmetrischen, linearen Dialkylethem können auch unsymmetrische Dialkylether zum Einsatz gelangen. Unsymmetrische Ether sind dadurch gekennzeichnet, daß die Alkylreste des Dialkylethers unterschiedlich sind. Hierzu gehören beispielsweise der n-Hexyl-n-octylether, n-Octyl-n-decylether, n-Decyl-n-undecylether, n-Undecyl-n-dodecylether und der n-Hexyl-n-Undecylether.

Die Alkylreste der Dialkylether können nicht nur linear und identisch oder verschieden sein, es können erfindungsgemäß ebenso Dialkylether eingesetzt werden, deren Alkylreste eine oder mehrere Verzweigungen aufweisen. Hierbei ist es unerheblich, ob die Verzweigungen in einem oder in beiden Alkylresten auftreten. Symmetrische Dialkylether mit verzweigten Alkylresten sind z.B. Di-tert-butylether, Di-iso-pentylether, Di-3-ethyldecylether. Beispiele für unsymmetrische Dialkylether mit der hier angesprochenen Struktur sind tert.-Butyl-n-octylether, iso-Pentyl-n-octylether oder 2-Methyl-pentyl-n-octylether.

Die Di-Alkylether können gegebenenfalls in den erfindungsgemäßen Zubereitungen in Mengen von 0,1 bis 5 Gew.-%, bevorzugt 0,5 bis 3 Gew.-%, vorhanden sein.

Eine weitere Substanzgruppe, die der erfindungsgemäßen Zubereitung vorteilhafte Eigenschaften in Bezug auf Pflegewirkung und kosmetischen Effekt verleiht, sind die Monoester der Fettsäuren mit Alkoholen mit 6 bis 24 C-Atomen. Bei dieser Stoffgruppe handelt es sich um die Produkte der Veresterung von Fettsäuren mit 8 bis 24 C-Atomen wie beispielsweise Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen, mit Alkoholen wie beispielsweise Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestem auf Basis von Fetten und Ölen oder Aldehyden aus der Roelen'schen Oxosynthese sowie als Monomerfraktion bei der Dimerisierung von ungesättigten Fettalkoholen anfallen. Die Fettsäuremonoester können in Mengen von 0,1 bis 5 Gew.-%, bevorzugt 0,8 bis 3 Gew.-%, bezogen auf die gesamte Zubereitung, in den Zubereitungen vorhanden sein.

Die erfindungsgemäßen Zubereitungen können grundsätzlich zur pflegenden Behandlung aller Fasern eingesetzt werden; besonderen Erfolg verspricht jedoch der Einsatz bei Fasern, die Naturprodukte darstellen oder aus einem solchen gewonnen werden. Insbesondere lassen sich keratinhaltige Fasern wie beispielsweise Wolle oder Seide erfolgreich behandeln. Die Anwendung der erfindungsgemäßen Zubereitungen ist jedoch nicht auf Textilfasern beschränkt, sondern eignet sich insbesondere auch zur Behandlung menschlicher Haare.

Zu diesem Zweck werden die erfindungsgemäßen Zubereitungen beispielsweise als sogenannte Haarspülung oder als Haarkur formuliert. Haarspülungen werden in der Regel so formuliert, daß ein Ausspülen der Wirkstoffe nach der gewünschten Einwirkzeit mit Wasser oder einem zumindest überwiegend wasserhaltigen Mittel vorgesehen ist. Die Kontaktzeit mit dem Haar ist in der Regel kurz. Haarkuren enthalten die Wirkstoffkombination in einer höheren Konzentration als Haarspülungen und sind zur Behandlung stark geschädigten Haares vorgesehen. Die Einwirkzeit kann kurz sein, beispielsweise in der Größenordnung der Einwirkzeit von Haarspülungen, sie kann jedoch auch je nach Grad der Schädigung des Haares bis zu 20 Minuten betragen. Auch die erfindungsgemäßen Haarkuren werden in der Regel nach Ablauf der Einwirkzeit mit Wasser oder einem zumindest überwiegend wasserhaltigen Mittel ausgespült, können jedoch auch auf dem Haar belassen werden. Die Anwendung dieser Zubereitungen bewirkt einen avivierenden Effekt am menschlichen Haar, der sich in einer verbesserten Naßkämmbarkeit, einer erhöhten Trockenkämmarbeit sowie einem guten Frisurenhalt äußert. Während die Verbesserung der Naßkämmbarkeit, d.h. eine Erniedrigung der Naßkämmarbeit, in allen Fällen gewünscht wird, sind die Verhältnisse bei der Trockenkämmbarkeit komplizierter. Niedrige Kämmarbeits-Werte charakterisieren zwar eine Verbesserung der Kämmbarkeit; wird die Kämmarbeit aber zu sehr erniedrigt, so verliert das Haar Fülle und Halt, so daß sich in Extremfall bestimmte Frisuren nicht mehr aufbauen lassen. Daher kann, vor allem bei komplexeren Frisuren, eine in bestimmten Grenzen erhöhte Trockenkämmarbeit durchaus erwünscht sein, um den Halt der Frisur zu verbessern.

Weiterhin können die erfindungsgemäßen Zubereitungen alle für den jeweiligen Verwendungszweck üblichen Zusätze enthalten. Insbesondere bei der Verwendung zur Haarbehandlung können dies kosmetische Zusätze sein, die eine reinigende, pflegende oder in anderer Weise das äußere Erscheinungsbild des Haares ändernde Wirkung entfalten. Auch solche Zusätze die keine direkte Einwirkung auf das Haar, ansonsten jedoch eine positive Wirkung auf die Kopfhaut oder beispielsweise die Haarfollikel entfalten, können in der erfindungsgemäßen Zubereitung enthalten sein.

Weitere übliche Bestandteile der erfindungsgemäßen Zubereitungen können sein:
- Anionische, zwitterionische, amphotere und nichtionische Polymere wie beispielsweise Vinylacetat/Crotonsäure-Copolymere, Polydimethylsiloxane,
   Vinylpyrrolidon/Vinylacrylat-Copolymere, Vinylacetat/Butylmaleat/Isobornyl-acrylat-Copolymere, Methylvinylether/Maleinsäureanhydrid-Copolymere und deren Ester, unvemetzte und mit Polyolen vemetzte Polyacrylsäuren, Acryl-amidopropyl-trimethylammoniumchlorid/Acrylat-Copolymere, Octylacrylamidl
   Methyl-methacrylat/tert.Butylaminoethylmethacrylat/2-Hydroxypropylmethacrylat-Copolymere, Polyvinylpyrrolidon, Vinylpyrrolidon/Vinylacetat-Copoly-mere, Vinylpyrrolidon/Dimethylaminoethylmethacrylat/Vinylcaprolactam-Terpolymere sowie gegebenfalls derivatisierte Celluloseether.
- Quarternierte Amine wie Methyl-1-alkylamidoethyl-2-alkylimidazoliniummethosulfat
- Verdickungsmittel wie Agar-Agar, Guar-Gum, Alginate und Xanthan-Gum,
- Strukturanten wie Glucose und Maleinsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle, Dimethylisosorbid und Cyclodextrine,
- Lösungsvermittler, wie Ethanol, Isopropanol, Ethylenglykol, Propylenglykol, Glycerin und Diethylenglykol,
- Farbstoffe,
- Antischuppenwirkstoffe wie Piroctone Olamine und Zink Omadine,
- weitere Substanzen zur Einstellung des pH-Wertes
- Wirkstoffe wie Panthenol, Allantoin, Pyrrolidoncarbonsäuren, Pflanzenextrakte und Vitamine,
- Lichtschutzmittel,
- Konsistenzgeber wie Zuckerester, Polyolester oder Polyolalkylether,
- Fette und Wachse, wie Walrat, Bienenwachs, Montanwachs und Paraffine,
- Fettsäurealkanolamide,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Ein weiterer Gegenstand der Erfindung ist die Verwendung einer wäßrigen Zubereitung zur avivierenden Faserbehandlung, dadurch gekennzeichnet, daß sie eine Wirkstoffkombination, bestehend aus 0,01 bis 30 Gew.-% Esterquats, 0,01 bis 30 Gew.-% Alkylamidoaminen und 0,01 bis 15 Gew.-% an Fettalkoholen mit 8 bis 22 C-Atomen, jeweils bezogen auf die gesamte Zusammensetzung, enthält.

Ebenfalls Gegenstand der Erfindung ist ein Verfahren zur pflegenden Behandlung von Fasern, indem die erfindungsgemäße Zubereitung in konzentrierter oder in verdünnter Form auf die Faser aufgebracht wird. Bevorzugt wird die erfindungsgemäße Zubereitung jedoch in der beschriebenen Form in einem Verfahren zur regenerierenden Behandlung menschlicher Haare eingesetzt.

Die folgenden Beispiele sollen den Gegenstand der Erfindung näher erläutern ohne ihn darauf einzuschränken.

### Beispiele

### 1. Beispielrezepturen

Alle Angaben verstehen sich als Gewichtsteile Aktivsubstanz

| **I. Haarspülungen** | | | |
|---|---|---|---|
| | | | INCI-Bezeichnung |
| (1) | Tego Amid® S18 | 1,20 | Stearamidopropyl-dimethylamine |
| | Foryl® 100 | 0,30 | Laureth-10 |
| | Lanette® O | 3,0 | Cetearyl Alkohol |
| | Dehyquart® AU 56 | 0,40 | Distearoylethylammonium Methosulfate |
| | Citronensäure | auf pH 3.5 | Citric Acid |
| | Wasser | ad 100 | |
| | | | |
| (2) | Tego Amid® D5040 | 1,60 | Cocamidopropyl-dimethylamine |
| | Cremephor® RH40 | 0,60 | PEG-40 Hydrogenated Castor Oil |
| | Lanette® O | 3,50 | |
| | Cutina® CP | 1,40 | Cetyl Palmitat |
| | Dehyquart® F75 | 0,50 | Distearoylethylammonium Methosulfate + Cetearyl Alcohol |
| | Konservierung | q.s. | |
| | Milchsäure | auf pH 3,5 | Lactic Acid |

| **II. Haarkuren** | | | |
|---|---|---|---|
| (3) | Tego Amid® S18 | 0,90 | |
| | Dehyquart® F 30 | 0,80 | Cetearyl Alcohol + Dipalmitoylethylammonium Methosulfate |
| | Tego Care® 450 | 0,30 | Polyglycerol Methylglucose Distearat |
| | Lanette® O | 5,20 | |
| | Cetiol® OE | 1.0 | Dicapryl Ether |
| | Eutanol® G | 0,80 | Octyldodecanol |
| | Konservierung | q.s. | |
| | Citronensäure | auf pH 3,5 | |
| | Wasser | ad 100 | |
| | | | |
| (4) | Emulgade® PL 1618 | 0,60 | Cetearyl Alcohol + Cetearyl Polyglucosid |
| | Mackine® 401 | 1.0 | Isostearamidopropyl Dimethylamine |
| | Dehyquart® AU46 | 0,50 | Dipalmitoylethylammonium Methosulfate |
| | DC® 200 | 0,80 | Dimethicone |
| | Rewoquat® W75 | 1.0 | Methyl-1-alkylamidoethyl-2-alkylimid-azolinium Methosulfat |
| | Lanette® O | 3.60 | |
| | Cutina® GMS | 0,60 | Glyceryl Stearate |
| | Chitosan | 0,2 | |
| | Konservierung | q.s. | |
| | Citronensäure | auf pH 3,5 | |
| | Wasser | ad 100 | |

### 2. Kämmbarkeitsuntersuchung

Die Wirksamkeit der erfindungsgemäßen Zubereitungen wurde anhand der folgenden Rezepturen untersucht:

| | A | B | C |
|---|---|---|---|
| Eumulgin® B2 | 0,40 | 0,40 | 0,40 |
| Stenol® 1618 | 3,0 | 3,0 | 3,0 |
| TEGO® Amid S18 | 1,0 | | 0,5 |
| Dehyquart® AU 46 | - | 1,11 | 0,55 |
| Citronensäure | 0,17 | - | 0,085 |

Anhand der Rezepturen wurden die Naßkämmarbeit sowie die Trockenkämmarbeit bestimmt.

**Tabelle 2:**

| Naßkämmarbeit | | | |
|---|---|---|---|
| Rezeptur | Naßkämmarbeit vorher [mJ] | Naßkämmarbeit nachher [mJ] | Differenz [%] |
| A | 62,2 | 17,5 | 72 |
| B | 57,3 | 16,3 | 72 |
| C | 60,1 | 16,4 | 73 |

**Tabelle 3:**

| Trockenkämmarbeit | | | |
|---|---|---|---|
| Rezeptur | Trockenkämmarbeit vorher [mJ] | Trockenkämmarbeit nachher [mJ] | Differenz [%] |
| A | 8,5 | 4,1 | 49 |
| B | 8,1 | 4,1 | 50 |
| C | 8,3 | 4,9 | 59 |

## Patentansprüche

1. Wäßrige Zubereitung zur avivierenden Faserbehandlung, **dadurch gekennzeichnet, daß** sie eine Wirkstoffkombination, bestehend aus 0,01 bis 30 Gew.-% Esterquats, 0,01 bis 30 Gew.-% Alkylamidoaminen und 0,01 bis 15 Gew.% an Fettalkoholen mit 8 bis 22 C - Atomen, jeweils bezogen auf die gesamte Zusammensetzung, enthält.

2. Zubereitung nach Anspruch 1, **dadurch gekennzeichnet, daß** die Wirkstoffkombination 0,1 bis 7 Gew.-% Esterquats zusammen mit 0,1 bis 7 Gew.-% Alkylamidoaminen enthält.

3. Zubereitung nach einem der Ansprüche 1 oder 2, **dadurch gekennzeichnet, daß** die Esterquats und die Alkylamidoamine in einem Gewichtsverhältnis von 20 zu 1 bis 1 zu 20, insbesondere von 10 zu 1 bis 1 zu 10, im Wirkstoffgemisch enthalten sind.

4. Zubereitung nach einem der Ansprüche 1 bis 3, **dadurch gekennzeichnet, daß** das Alkylamidoamin einen linearen Fettsäurerest mit 8 bis 22 C-Atomen, bevorzugt mit 14 bis 20 C-Atomen aufweist.

5. Zubereitung nach einem der Ansprüche 1 bis 4, **dadurch gekennzeichnet, daß** sie einen pH-Wert zwischen 2 und 7, insbesondere von 2,5 bis 5,5 aufweist.

6. Zubereitung nach einem der Ansprüche 1 bis 5, **dadurch gekennzeichnet, daß** sie als Haarspülung formuliert ist.

7. Zubereitung nach einem der Ansprüche 1 bis 6, **dadurch gekennzeichnet, daß** sie als Haarkur formuliert ist.

8. Verwendung einer wäßrigen Zubereitung nach einem der Ansprüche 1 bis 7 zur avivierenden Faserbehandlung.

9. Verwendung nach Anspruch 8, **dadurch gekennzeichnet, daß** die Zubereitung zur Behandlung von Fasern keratinischer, insbesondere menschlicher, Natur eingesetzt wird.

10. Verfahren zur regenerierenden Behandlung menschlicher Haare, **dadurch gekennzeichnet, daß** eine Zubereitung nach einem der Ansprüche 1 bis 7 auf das Haar aufgebracht wird.

## Claims

1. An aqueous preparation for softening fiber treatment, **characterized in that** it contains a combination of active ingredients consisting of 0.01 to 30% by weight esterquats, 0.01 to 30% by weight alkyl amidoamines and 0.01 to 15% by weight C₈₋₂₂ fatty alcohols, based on the composition as a whole.

2. A preparation as claimed in claim 1, **characterized in that** the combination of active ingredients contains 0.1 to 7% by weight esterquats together with 0.1 to 7% by weight alkyl amidoamines.

3. A preparation as claimed in claim 1 or 2, **characterized in that** the esterquats and the alkyl amidoamines are present in the combination in a ratio by weight of 20:1 to 1:20 and more particularly 10:1 to 1:10.

4. A preparation as claimed in any of claims 1 to 3, **characterized in that** the alkylamidoamine contains a linear fatty acid residue containing 8 to 22 and preferably 14 to 20 carbon atoms.

5. A preparation as claimed in any of claims 1 to 4, **characterized in that** it has a pH value of 2 to 7 and more particularly 2.5 to 5.5.

6. A preparation as claimed in any of claims 1 to 5, **characterized in that** it is formulated as a hair rinse.

7. A preparation as claimed in any of claims 1 to 6, **characterized in that** it is formulated as a hair conditioner.

8. The use of the aqueous preparation claimed in any of claims 1 to 7 for the softening treatment of fibers.

9. The use claimed in claim 8, **characterized in that** the preparation is used for the treatment of fibers of a keratinous, more particularly human, nature.

10. A process for the regenerating treatment of human hair, **characterized in that** the preparation claimed in any of claims 1 to 7 is applied to the hair.

## Revendications

1. Préparation aqueuse pour le traitement des fibres par avivage,
**caractérisée en ce qu'**
elle contient une combinaison de substances actives constituée de 0,01 à 30% en poids d'esterquats, de 0,01 à 30% en poids d'alkylamidoamines et de 0,01 à 15 % en poids d'alcools gras comportant de 8 à 22 atomes de carbone, toujours par rapport à l'ensemble de la composition.

2. Préparation selon la revendication 1,
**caractérisée en ce que**
la combinaison de substances actives contient de 0,1 à 7 % en poids d'esterquats avec de 0,1 à 7 % en poids d'alkylamidoamines.

3. Préparation selon l'une des revendications 1 ou 2,
**caractérisée en ce que**
les esterquats et les alkylamidoamines sont contenus dans le mélange de substances actives dans un rapport pondéral de 20 : 1 à 1 :20, en particulier de 10 :1 à 1 :10.

4. Préparation selon l'une des revendications 1 à 3,
**caractérisée en ce que**
l'alkylamidoamine présente un radical acide gras linéaire comportant de 8 à 22 atomes de carbone, de préférence de 14 à 20 atomes de carbone.

5. Préparation selon l'une des revendications 1 à 4,
**caractérisée en ce qu'**
elle présente un pH compris entre 2 et 7, en particulier entre 2,5 et 5,5.

6. Préparation selon l'une des revendications 1 à 5,
**caractérisée en ce qu'**
elle est formulée comme produit de rinçage des cheveux.

7. Préparation selon l'une des revendications 1 à 6,
**caractérisée en ce qu'**
elle est formulée comme produit de soin des cheveux.

8. Utilisation d'une préparation aqueuse selon l'une des revendications 1 à 7 pour le traitement des fibres par avivage.

9. Utilisation selon la revendication 8,
**caractérisée en ce que**
la préparation est utilisée pour le traitement des fibres de nature kératinique, en particulier humaines.

10. Procédé de traitement par régénération des cheveux humains, **caractérisé en ce qu'**
on dépose sur les cheveux une préparation selon l'une des revendications 1 à 7.
